(19) Europäisches Patentamt
European Patent Office
Office européen des brevets

(11) **EP 2 974 599 A1**

(12) # EUROPEAN PATENT APPLICATION
published in accordance with Art. 153(4) EPC

(43) Date of publication:
**20.01.2016 Bulletin 2016/03**

(21) Application number: **13878010.1**

(22) Date of filing: **11.03.2013**

(51) Int Cl.:
*A01N 63/00* *(2006.01)*     *A01P 3/00* *(2006.01)*
*A01G 7/00* *(2006.01)*     *C12N 1/20* *(2006.01)*

(86) International application number:
**PCT/JP2013/056640**

(87) International publication number:
**WO 2014/141362 (18.09.2014 Gazette 2014/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(83) **Declaration under Rule 32(1) EPC (expert solution)**

(71) Applicants:
• **New Environmental Technology Council Tokyo 102-0094 (JP)**

• **Tokyo University of Agriculture Tokyo 156-8502 (JP)**

(72) Inventors:
• **SHINOHARA Hirosuke Atsugi-shi Kanagawa 243-0034 (JP)**
• **HIROSE Yoichiro Tokyo 105-0011 (JP)**

(74) Representative: **Novagraaf Technologies Bâtiment O2 2, rue Sarah Bernhardt CS90017 92665 Asnières-sur-Seine Cedex (FR)**

(54) **NOVEL COLLIMONAS BACTERIA AND METHOD FOR CONTROLLING HARMFUL PLANT PATHOGEN USING SAID BACTERIA**

(57)     The objective of the present invention is to provide a means for imparting control to agriculturally useful plants against diseases caused by a pathogenic filamentous fungus, pathogenic bacteria or pathogenic virus.

The present invention relates to: a method for controlling plant diseases using a bacteria that belongs to the genus Collimonas and controls diseases caused by a pathogenic filamentous fungus, pathogenic bacteria or pathogenic virus; and a plant created from said method.

EP 2 974 599 A1

**Description**

Technical Field

The present invention relates to a novel Collimonas bacterium, and to a method for controlling a harmful plant pathogen by using the bacterium.

Background Art

The conventional technologies for controlling plant pathogenic bacteria caused by pathogenic filamentous fungi, pathogenic bacteria or pathogenic viruses by mainly chemical agrochemicals have contributed to efficient food security. However, environment preservation-type agriculture using no pesticide or a decreased amount of pesticide including not only the efficiency of culturing but also reassurance and safeness has been demanded in recent years, and thus a technology for controlling a plant pathogenic bacterium (such as a microbial agrochemical) which conform to such demand is required.

Various agrochemicals having special chemical ingredients for controlling these plant pathogenic bacteria have been considered and suggested, and other approaches that are different from chemical agrochemicals have been considered; for example, studies for suppressing the proliferation of vegetable pathogenic bacteria by using Collimonas bacteria have been conducted (Non-patent Literatures 1 to 4).

Prior Art Document

Non-patent Literature

Non-patent Literature 1 : Wieste de Boer, Johan H. J. Leveau, George A. Kowalchuk, Paulien J. A. Klein Gunnewiek, Edwin C. A. Abeln, Marian J. Figge, Klaas Sjollema, Jaap D. Janse and Johannes A. van Veen: Collimonas fungivorans gen. nov., sp. nov., a chitinolytic soil bacterium with the ability to grow on living fungal hyphae.

Non-patent Literature 2: Francesca Mela, Kathrin Fritsche, Wietse de Boer, Johannes A van Veen, Leo H de Graaff, Marlies van den Berg and Johan HJ Leveau: Dual transcriptional profiling of a bacterial/fungal confrontation: Collimonas fungivorans versus Aspergillus niger

Non-patent Literature 3: Faina Kamilova, Johan H. J. Leveau and Ben Lugtenberg: Collimonas fungivorans, an unpredicted in vitro but efficient in vivo biocontrol agent for the suppression of tomato foot and root rot

Non-patent Literature 4: Sachie Hoppener-Ogawa: Ecology of mycophagous Collimonas bacteria in soi

Summary of Invention

Technical Problem

Studies for suppressing the proliferation of vegetable pathogenic bacteria by Collimonas bacteria as mentioned above have been reported, but any useful report on a Collimonas bacterium that controls all vegetable pathogenic bacteria including pathogenic filamentous fungi, pathogenic bacteria or pathogenic viruses has not been made yet.

Therefore, the present invention aims at providing a novel Collimonas bacterium that controls a pathogenic filamentous fungus, a pathogenic bacterium or a pathogenic virus in a plant, and a method for controlling a harmful plant pathogen by using the bacterium.

Solution to Problem

In order to solve the above-mentioned problem, the inventors have considered whether or not a pathogenic filamentous fungus, a pathogenic bacterium or a pathogenic virus in a plant can be controlled, by using a Collimonas bacterium, D-25 strain, which was deposited with the accession number NITE P-1104 with the Patent Microorganisms Depositary of the National Institute of Technology and Evaluation (2-5-8, Kazusakamatari, Kisarazu-shi, Chiba, Japan) (hereinafter referred to as "D-25 strain") on June 9, 2011.

Consequently, the inventors found that a pathogenic filamentous fungus, a pathogenic bacterium or a pathogenic virus in a plant can be controlled, and attained the present invention.

Specifically, in order to solve the above-mentioned problem, the following inventions are suggested.

The invention of claim 1 is a method for controlling a disease damage by a pathogenic filamentous fungus, a pathogenic bacterium or a pathogenic virus in a plant, including a step of artificially infecting a plant with a bacterium that belongs to the genus Collimonas and has an ability to impart a resistance against a disease damage by a pathogenic filamentous fungus, a pathogenic bacterium or a pathogenic virus to a host plant by living in symbiosis in the body of the plant.

The invention of claim 2 is the method for controlling a disease damage by a pathogenic filamentous fungus, a pathogenic bacterium or a pathogenic virus in a plant according to claim 1, wherein the bacterium is a novel Collimonas bacterium (Accession No. NITE P-1104).

The invention of claim 3 is the method for controlling a disease damage by a pathogenic filamentous fungus, a pathogenic bacterium or a pathogenic virus in a plant according to claim 1 or 2, wherein the plant is a plant that belongs to Gramineae or Solanaceae.

The invention of claim 4 is an agent for controlling a disease damage by a pathogenic filamentous fungus, a pathogenic bacterium or a pathogenic virus in a plant, which contains, as an active ingredient, a bacterium that belongs to the genus Collimonas and has an ability to impart a resistance against a disease damage by a pathogenic filamentous fungus, a pathogenic bacterium or a pathogenic virus to a host plant by living in symbiosis in the body of the plant.

The invention of claim 5 is the controlling agent according to claim 4, wherein the bacterium is a novel Collimonas bacterium (Accession No. NITE P-1104).

The invention of claim 6 is the controlling agent according to claim 4 or 5, wherein the plant is a plant that belongs to Gramineae or Solanaceae.

The invention of claim 7 is a plant having a resistance against a disease damage by a pathogenic filamentous fungus, a pathogenic bacterium or a pathogenic virus, which has been artificially infected with a novel Collimonas bacterium (Accession No. NITE P-1104) that has an ability to impart a resistance against a disease damage by a pathogenic filamentous fungus, a pathogenic bacterium or a pathogenic virus to a host plant by living in symbiosis in the body of the plant.

Advantageous Effects of Invention

According to this invention, a novel Collimonas bacterium that controls a pathogenic filamentous fungus, a pathogenic bacterium or a pathogenic virus in a plant, and a method for controlling a harmful plant pathogen using this bacterium can be provided.

Brief Description of Drawings

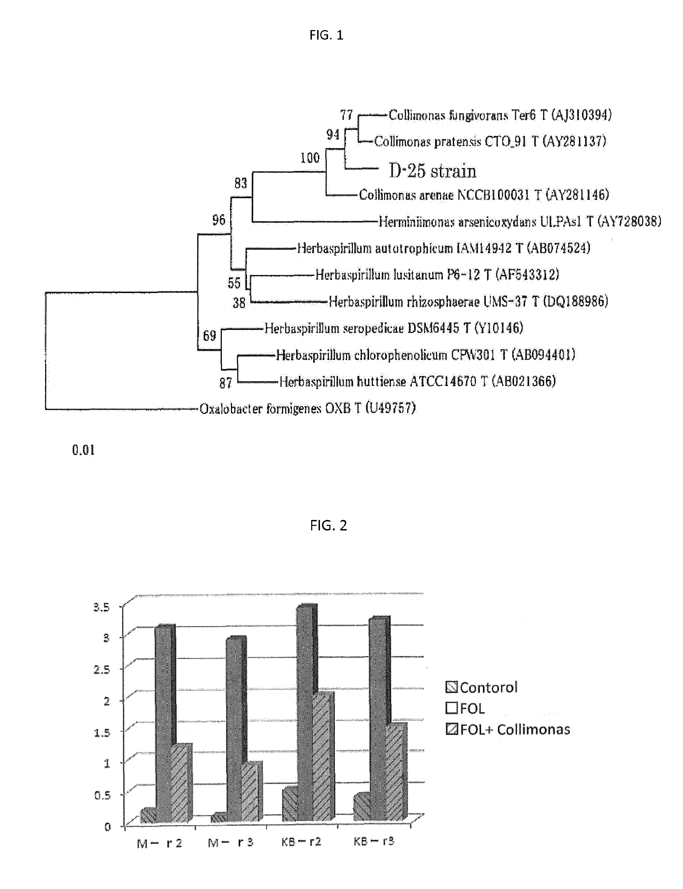

Fig. 1 is a drawing showing the molecular phylogenetic systematics of D-25 strain based on the 16s rRNA gene sequence.

Fig. 2 shows a drawing showing the suppression effects of Solanaceae plant samples to which D-25 strain has been seeded on tomato wilt disease (in the drawing, the left side of each graph represents a control (not seeded), the middle side represents a sample to which D-25 strain has not been seeded, and the right side represents a sample to which D-25 strain has been seeded).

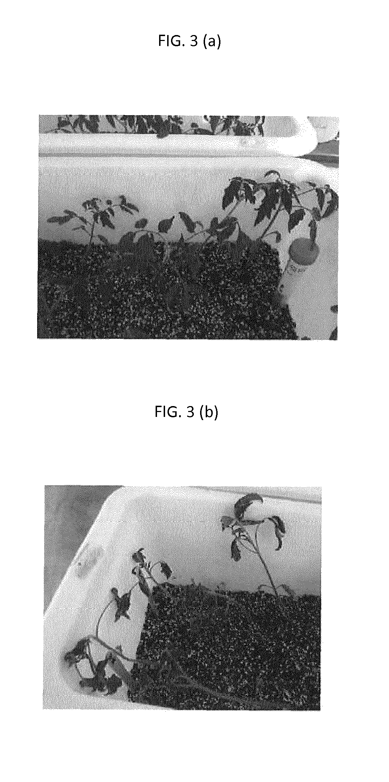

Figs. 3(a) and 3(b) show pictures that represent the effect of suppressing tomato wilt disease of a Solanaceae plant sample to which D-25 strain has been seeded, in which Fig. 3(a) represents a sample to which D-25 strain has been seeded, and Fig. 3(b) represents a sample to which D-25 strain has not been seeded.

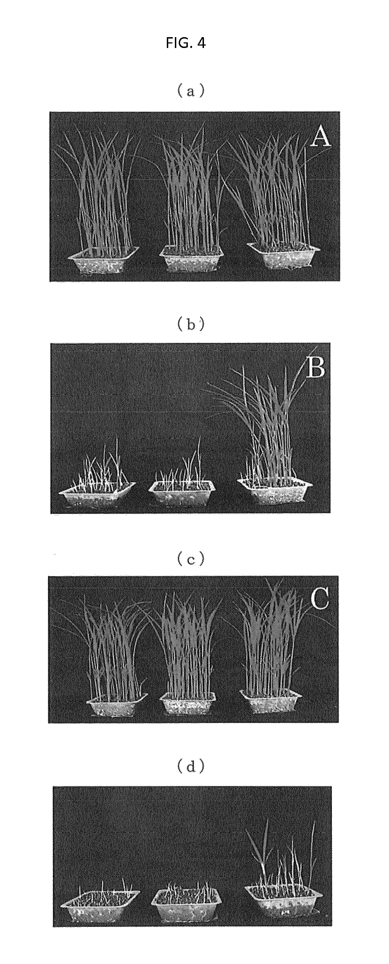

Figs. 4(a) to 4(d) show pictures that represent the effect of suppressing rice bacterial grain rot on a seed rice sample to which D-25 strain has been seeded, in which Fig. 4(a) represents healthy rice seedlings, Fig. 4(b) represents a drawing in which a culture supernatant liquid of D-25 is used, Fig. 4(c) represents a drawing in which a fungus body suspension liquid of D-25 strain is used, and Fig. 4(d) represents a sample to which D-25 strain has not been seeded.

Description of Embodiments

(Mycological properties of D-25 strain)

The mycological properties of D-25 strain in the present invention are as follows.

(1) Molecular phylogenetic analysis of D-25 strain

The classification and identification of D-25 strain were conducted by a molecular phylogenetic analysis based on the 16S rRNA gene sequence.

The extraction of DNA from a fungus body (bacterium) grown on an R2A culture medium was conducted by using ISOIL for Beads Beating (manufactured by Nippon Gene Co., Ltd.). A cultured fungus body was collected in a dedicated plastic tube with a volume of 2 mL, and 950 $\mu$L of Lysis Solution BB and 50 $\mu$L of Lysis Solution 20S were added thereto. The tube was then vigorously stirred by using a bead beater, and subjected to centrifugation (12,000 x g, for 1 min, at room temperature). After the centrifugation, 600 $\mu$L of the supernatant was transferred to a new tube, 400 $\mu$L of Purification Solution was added thereto, and the mixture was sufficiently mixed. 600 $\mu$L of chloroform was then added, and the mixture was mixed and centrifuged (12,000 x g, for 15 min, at room temperature). After the centrifugation, 800 $\mu$L of the aqueous phase was transferred to a new tube, 800 $\mu$L of Precipitation Solution was added thereto, and the mixture was sufficiently mixed and centrifuged (20,000 x g, for 15 min, 4°C) . The supernatant was discarded, 1 mL of 70% ethanol was added, and the mixture was sufficiently mixed and centrifuged (20,000 x g, 5 min, 4°C) . The supernatant was then discarded, air drying was conducted, and the precipitate was dissolved in 50 $\mu$L of a TE buffer solution (pH 8.0).

The extracted genome DNA was subjected to PCR amplification by using universal primers 27f and 1492r (the primers target the 16S rRNA gene region of the bacterium) (Table 1). The PCR was conducted at a reaction capacity of 20 $\mu$L by using Thermal Cycler 2720 (Applied Biosystems). A reaction solution was prepared by PCR enzyme TaKaRa Ex Taq (TaKaRa) and an accompanying PCR reagent. The composition per 20 $\mu$L of the reaction solution was 14.7 $\mu$L of sterilized water, 2 $\mu$L of the buffer solution, 1 $\mu$L (< 1 ng) of the genome DNA, 10 pmol/L of each primer (0.8 $\mu$L each), 1.6 $\mu$L of a dNTP solution and 0.1 $\mu$L (0.025 U) of Ex Taq. In addition, a reaction solution using E. coli genome as a template DNA was used as a positive control, and a reaction solution to which any template DNA solution had not been added was used as a negative control. 30 cycles of temperature cycling were conducted, in which one cycle included initial denaturation at 94°C for 1 min, thermal denaturation at 94°C for 30 sec, annealing at 55°C for 30 sec, and an extension reaction at 72°C for 90 sec. After the PCR, 5 $\mu$L of the reaction solution was collected and analyzed by 1.5% agarose gel electrophoresis. The detection was conducted by staining an agarose gel with ethidium bromide and using a UV illuminator (UVP).

The sequencing analysis of the obtained PCR fragment (about 1,500 bp) was conducted. For the sequencing reaction, primers 27f, 519f, 1099f, 520r and 1492r were used (Table 3), and the base sequence was determined by an ABI Prism 3100 Genetic Analyzer (Applied Biosystems). For the result of the base sequence, a homology search by BLAST was conducted at GenBank. Multiple alignments were conducted by using CLUSTAL W together with the sequences for which homology was shown, and a molecular genealogical tree was prepared by a neighbor-joining method by using MEGA4.0.

[Table 1]

Oligonucleotide primers for bacterial 16S rRNA gene analysis used in test

| Primer | Base sequence |
| --- | --- |
| 27f | 5'-AGAGTTTGATCCTGGCTCAG-3' |
| 519f | 5'-CAGCMGCCGCGGTAATWC-3' |
| 1099f | 5'-GYAACGAGCGCAACCC-3' |
| 520r | 5'-ACCGCGGCTGCTGGC-3' |
| 1492r | 5'-ACGGYTACCTTGTTACGACTT-3' |

When a classification was conducted on D-25 strain based on the base sequence, it was clarified that the D-25 strain had high homology with the base sequences of Collimonas bacteria. Table 2 shows the results of the homology search on D-25 strain, and Fig. 1 shows the molecular genealogical tree.

[Table 2]

| Presumption of related species of D-25 strain (results of homology search by BLAST search) | |
| --- | --- |
| BLAST search results (Top 5 with high homology) | Homology score |
| *Collimonas pratensis* CTO291 | 99.5 % |
| *Collimonas* sp. III-47 | 99.2 % |
| *Collimonas* sp. III-32 | 99.2 % |
| *Collimonas* sp. III-15 | 99.2 % |
| *Collimonas* sp. III-5 | 99.2 % |
| *Collimonas fungivorans* CTE118 | 99.4 % |

(2) Physiological and biochemical tests on D-25 strain

The results of the physiological and biochemical tests on D-25 strain are as shown in Tables 3, 4 and 5.

[Table 3]

| Results of physiological and biochemical tests on D-25 strain (form, motility, growth temperature tests, etc.) | |
|---|---|
| Test item | Test result |
| Culture temperature | 25 °C |
| Cell form | Bacillus, extended type is present (0.9-1.0 x 2.0-4.0 $\mu$m) |
| Gram stainability | - |
| Presence or absence of spore | - |
| Motility | - |
| Colony form | Culture medium: R2A agar<br>Culture time: 48 hours<br>Diameter: 2.0-3.0mm<br>Color tone: pale yellow<br>Shape: circular shape<br>State of projection: lens-shape<br>Periphery: Wave shape<br>Shape of surface, and the like: smooth<br>Transparency: translucent<br>Viscosity: viscous |
| Growth temperature test<br>30°C | +<br> |
| 37°C | - |
| Catalase reaction | + |
| Oxidase reaction | + |
| Generation of acid/gas from glucose | -/- |
| O/F test (oxidation/fermentation) | -/- |
| Growth under anaerobic condition | - |
| +: positive, -: negative | |

[Table 4]

| Results of physiological and biochemical tests on D-25 strain (biochemical tests, assimilation tests) | | | |
|---|---|---|---|
| Test item | Judgment | Test item | Judgment |
| Nitrate reduction* | - | D-mannitol** | + |
| Indole production* | - | N-acetyl-D-glucosamine** | + |
| Glucose acidification* | - | Maltose** | - |
| Arginine dihydrolase* | - | Potassium gluconate** | + |
| Urease* | - | n-capric acid** | - |
| Esculin hydrolysis* | - | Adipic acid** | - |
| Gelatin hydrolysis* | - | dl-malic acid** | + |
| β-Galactosidase* | - | Sodium citrate** | + |
| Glucose** | + | Phenyl acetate** | - |
| L-arabinose** | + | Cytochrome oxidase* | - |

(continued)

| Results of physiological and biochemical tests on D-25 strain (biochemical tests, assimilation tests) | | | |
|---|---|---|---|
| Test item | Judgment | Test item | Judgment |
| D-mannose** | + | | |
| *biochemical test, **assimilation test<br>+: positive, -: negative | | | |

[Table 5]

| Results of physiological and biochemical tests on D-25 strain (enzyme reaction tests) | |
|---|---|
| Test item | Test result |
| Alkali phosphatase | + |
| Esterase (C4) | + |
| Esterase lipase (C8) | + |
| Lipase (C14) | + |
| Leucine allylamidase | + |
| Valine allylamidase | - |
| Cystine allylamidase | - |
| Tripsin | - |
| Chymotripsin | - |
| Acidic phosphatase | + |
| Naphthol-AS-B1-phosphohydrolase | + |
| $\alpha$-Galactosidase | - |
| $\beta$-Galactosidase | + |
| $\beta$-Glucuronidase | - |
| $\alpha$-Glucosidase | - |
| $\beta$-Glucosidase | - |
| N-Acetyl-$\beta$-glucosaminidase | - |
| $\alpha$-Mannosidase | - |
| $\alpha$-Fucosidase | - |
| +: positive, -: negative | |

(3) Discussion

Since the 16S rRNA gene sequence of D-25 strain corresponded to those of the Collimonas bacteria by 99% or more and D-25 strain was contained in the genealogical tree of the genus Collimonas in the molecular genealogical tree, it is conjectured that D-25 strain belongs to this genus.

Secondly, D-25 strain was a gram negative bacillus having no motility, formed a viscous colony on the R2A agar culture medium, did not grow under an anaerobic condition, did not oxidize glucose, and showed positive in both of the catalase reaction and the oxidase reaction (Table 3).

Furthermore, as the results of the physiological and biochemical tests, D-25 strain did not reduce a nitrate salt, did not produce indole, showed no arginine dihydrolase activity, assimilated glucose, L-arabinose and D-mannitol and the like, and did not assimilate n-capric acid and phenyl acetate and the like (Table 4).

Furthermore, as the test results of the enzyme reactions, D-25 strain showed activities for alkali phosphatase, esterase (C4) and esterase lipase (C8) and the like, and did not show activities for valine allyl amidase, $\alpha$-galactosidase and the like (Table 5).

In these characteristics, many similarities to the already-known species of the genus Collimonas, for which attribution was conjectured from the results of the phylogenetic analysis based on 16S rRNA gene sequence, were recognized. However, these characteristics were different from the characteristics of the already-known species of the genus Collimonas in that motility was not shown (Table 3) and a valine allyl amidase activity was not shown (Table 5).

From the above-mentioned mycological properties, it was presumed that D-25 strain is a novel Collimonas bacterium that belongs to the genus Collimonas taxon. This strain was deposited with the accession number NITE P-1104 with the Patent Microorganisms Depositary of the National Institute of Technology and Evaluation (2-5-8, Kazusakamatari, Kisarazu-shi, Chiba, Japan) on June 9, 2011.

Examples of the plant to which a resistance against a disease damage by a pathogenic filamentous fungus, a pathogenic bacterium or a pathogenic virus is imparted by infecting with the bacterium of the present invention include Gramineae plants, Brassicaceae plants, Solanaceae plants, Asteraceae plants, Alliaceae plants or Cucurbitaceae plant.

The Gramineae plants especially include grains such as rice, wheat, barley, rye, rye wheat, pearl barley, sorghum, oat, corn, sugar cane, foxtail millet and Japanese millet. The Gramineae plants further include feedstuff or pasture plants such as lawn grass, buffalo grass, Bermuda grass, weeping grass, centipede grass, carpet grass, Dalis grass, Kikuyu grass and St. Augustine grass.

The Brassicaceae plants especially include rape, turnip, qing-geng-cai, nozawana, mustard, takana, Chinese mustard, potherb mustard, kohlrabi, rucola, watercress, tatsoi, cauliflower, cabbage, kale, Chinese cabbage, Japanese mustard spinach, Japanese radish, radish, broccoli, brussels sprouts, Japanese horseradish and horseradish.

The Solanaceae plants include eggplant, tomato, potato, red pepper, pepper and paprika.

The Asteraceae plants include lettuce and Chop-suey greens.

The Alliaceae plants include onion, green onion, Chinese chive, Chinese onion and garlic.

The Cucurbitaceae plants include cucumber, melon, watermelon and pumpkin.

The present invention further relates to the above-mentioned plants artificially infected with the bacterium of the present invention, which have resistance against a disease damage by a pathogenic filamentous fungus, a pathogenic bacterium or a pathogenic virus.

Examples of the plant disease damage by a pathogenic filamentous fungus which can be controlled by the present invention include rice blast (pathogenic filamentous fungus: Magnaporthe grisea), rice brown spot (pathogenic filamentous fungus: Bipolaris leersiae), rice bakanae disease (pathogenic filamentous fungus: Gibberella fujikuroi), rice sheath blight (pathogenic filamentous fungus: Thanatephorus cucumuris), rice downy mildew fungus (pathogenic filamentous fungus: Ssclerophthora macrospora), rice pseudo sheath blight (pathogenic filamentous fungus: Rhizoctonia solani), wheat ergot (pathogenic filamentous fungus: Claviceps purpurea), wheat loose smut (pathogenic filamentous fungus: Ustilago tritici), barley loose smut (pathogenic filamentous fungus: Ustilago nuda), rye typhula snow blight (pathogenic filamentous fungus: Typhula incarnata), rye leaf spot (pathogenic filamentous fungus: Cochliobolus sativus), damping-off of rice, oat, wheat, barley and rye (pathogenic filamentous fungus: Gaeumannomyces graminis), corn glume mold (pathogenic filamentous fungus: Setosphaeria turcica), clubroot of Brassicaceae vegetables (pathogenic filamentous fungus: Plamodiophora brassicae), damping-off of Brassicaceae vegetables (pathogenic filamentous fungus: Thanatephorus cucumeris), Chinese cabbage yellow (pathogenic filamentous fungus: Verticillium albo-atrum), radish chlorosis (pathogenic filamentous fungus: Fusarium oxysporum f. sp. Raphani), radish white rust (pathogenic filamentous fungus: Albugo macrospora), Japanese mustard spinach white rust (pathogenic filamentous fungus: Albugo macrospora), cucumber fusarium wilt (pathogenic filamentous fungus: Fusarium oxysporum Schlechtendahl f. sp. cucumerinum Owen), melon fusarium wilt (pathogenic filamentous fungus: Fusarium oxysporum Schlechtendahl: Fries f. sp. melonis (Leach et Currence) Snyder et Hansen), tomato wilt disease (pathogenic filamentous fungus: Fusarium oxysporum Schlechtendahl: Fries f. sp. lycoperisici (Saccardo) Snyder & Hansen) and cucumber powdery mildew (pathogenic filamentous fungus: Sphaerotheca cucurbitae (Jaczewski) Zhao).

Examples of the plant disease damage by a pathogenic bacterium which can be controlled by the present invention include rice bacterial leaf blight (pathogenic bacterium: Xanthomonas oryzae pv. oryzae), rice bacterial grain rot (pathogenic bacterium: Pseudomonas glumae), vegetable bacterial soft rot which leads to serious damages on Chinese cabbage and Brassicaceae vegetables (pathogenic bacterium: Erwinia carotovora), cabbage black rot (Xanthomonas campestris pv. campestris) and rice bacterial brown stripe (pathogenic bacterium: Pseudomonus avenae Manns 1909).

The Examples mentioned below indicate that the bacterium according to the present invention is effective for controlling a disease damage in a plant by a pathogenic filamentous fungus, and is effective for controlling a plant disease damage by a pathogenic bacterium. Accordingly, it is understood that the bacterium according to the present invention controls the disease damage of the host plant itself. Accordingly, the bacterium according to the present invention is effective for not only the control of a plant disease damage by a pathogenic filamentous fungus or a pathogenic bacterium, but also the control of a plant disease damage by a pathogenic filamentous fungus, a pathogenic bacterium or a pathogenic virus.

Examples of the plant disease damage by a pathogenic virus which can be controlled by the present invention include rice dwarf Rice dwarf reovirus, rice stripe Rice stripe tenuivirus, rice black-streaked dwarf Rice blach-streaked dwarf reovirus, rice necrosis mosaic Rice necrosis mosaic potyvirus, rice waika Rice waika virus, wheat yellow mosaic Wheat yellow mosaic virus, barley yellow mosaic Barley yellow mosaic virus, barley stripe mosaic virus Barley stripe hordeivirus, and viral diseases of radish, turnip and Japanese mustard spinach including cucumber mosaic virus, turnip mosaic potyvirus, radish enation mosaic comovirus and broad bean wilt fabavirus.

The bacterium that can be used in the present invention is not especially limited as long as it is a bacterium that belongs to the genus Collimonas and has an ability to impart a resistance against a disease damage by a pathogenic filamentous fungus, a pathogenic bacterium or a pathogenic virus to a host plant by living in symbiosis in the body of the plant. Specifically, a novel Collimonas bacterium (Accession No. NITE P-1104) is exemplified.

The bacterium used in the present invention can be cultured under general conditions by a general culture process such as shaking culture. Examples of the culture medium used for culturing include synthetic or natural culture media each containing a sugar such as glucose, sucrose, starch or dextrin as a carbon source; an ammonium salt such as ammonium sulfate, ammonium chloride or ammonium nitrate, an inorganic nitrogen source such as a nitrate salt, or an organic nitrogen source such as a yeast extract, corn steep liquor, a meat extract, wheat germ, polypepton, sugar cane strained lees (bagasse), beer lees, a soybean powder, rice bran or a fish powder, as a nitrogen source; and a salt containing phosphorus, potassium, manganese, magnesium, iron or the like such as monopotassium phosphate, magnesium sulfate, manganese sulfate or ferrous sulfate as an inorganic salt.

Furthermore, the present invention relates to an agent for controlling a disease damage by a pathogenic filamentous fungus, a pathogenic bacterium or a pathogenic virus in a plant, which contains the bacterium of the present invention as an active ingredient. As the plant disease damage controlling agent, the culture liquid of the bacterium of the present invention can be directly used, or a high-concentration product of the present invention formed by separating the culture liquid of the bacterium by a method such as film separation, centrifugation or filtration separation can also be used.

Furthermore, as the plant disease damage controlling agent of the present invention, a product formed by drying the culture liquid of the bacterium of the present invention can be used. Alternatively, a product formed by adsorbing the culture liquid of the bacterium of the present invention with a porous adsorbent such as an active carbon powder, diatomite or talc, and drying the adsorbent can be used. The drying method may be a general method, and may be freeze drying or drying under a reduced pressure. These dried products may further be pulverized by a pulverization means such as a ball mill after the drying.

The bacterium of the present invention itself can be used singly in the present invention as the above-mentioned culture liquid, high concentration product or dried product, and may also be provided as a composition for controlling a plant disease damage by combining with other arbitrary ingredients and forming into a formulation having a similar form to that of a general microorganism formulation (for example, forms such as a powdery agent, a hydrate agent, an emulsion agent, a liquid agent, a flowable agent or an application agent). The arbitrary ingredients that can be used in combination include materials that are allowed to be applied to plants such as a solid support and an auxiliary agent.

It is preferable that a plant is infected with the bacterium of the present invention in the vegetative and growth periods of the plant.

As the method for applying the bacterium of the present invention or a composition containing the bacterium to a plant, spraying, perfusion, dipping, application to a plant body, contacting with an artificially-formed scratch, injection by a syringe, mixing with a soil, mixing into a water culture medium, a method in which the bacterium is mixed with sand or the like and blowing the mixture as in sand blasting, and the like are considered. In the case when a plant is subjected to a perfusion treatment with a suspension liquid formed by suspending the bacterium of the present invention, the concentration of the bacterium of the present invention in the suspension liquid is preferably from $10^4$ to $10^{12}$ CFU/ml.

Example 1

This Example shows the effect of D-25 strain to suppress pathogenesis on tomato wilt disease (F. oxysporum f. sp. lycoperisci Race 1).

(Experimental method)

As Solanaceae plant samples, Tomato CV. Momotaro and KyouryokuBeiji were used.

D-25 strain (1 x $10^8$/plant) that has been cultured in a wheat bran or rice bran culture medium is mixed with a soil for each of the above-mentioned samples in a seedling raising pot, and seeds are sown thereon.

When about three true leaves have developed, the plant is transferred to a pathogenic bacterium-contamination soil, and a pathogenic bacterium is seeded on the plant. At 2 to 4 weeks after the seeding of the pathogenic bacterium, the disease symptom of each sample is evaluated. In the evaluation method, the evaluation is conducted by imparting an index for each degree of pathogenesis (healthy seedling: 0, at critical region of pathogenesis: 1, light pathogenesis in seedling: 2, heavy pathogenesis in seedling: 3, withered seedling: 4).

Figs. 2 to 3(b) showed the effect of D-25 strain to suppress the pathogenesis on tomato wilt disease (F. oxysporum f. sp. lycoperisci).

As shown in Fig. 2, among the above-mentioned indices, an evaluation of around 1 was obtained in the Tomato CV. Momotaro to which D-25 strain had been seeded. Therefore, the effect of D-25 strain to suppress the pathogenesis on tomato wilt disease (F. oxysporum f. sp. lycoperisci) was confirmed (M-r2 and M-r3 in Fig. 2, and Fig. 3(a)).

Furthermore, among the above-mentioned indices, an evaluation of 1.5 to 2.0 was obtained for the KyouryokuBeiji to which D-25 strain had been seeded. Therefore, the effect of D-25 strain to suppress pathogenesis on tomato wilt disease (F. oxysporum f. sp. lycoperisci) was confirmed (KB-r2 and KB-r3 in Fig. 2).

Example 2

This Example shows the effect of D-25 strain to suppress the pathogenesis on a rice bacterial grain rot bacterium (Burkholderia glumae MAFF301441).

(Experimental method)

Healthy seed rice (breed: Koshihikari) was immersed in a suspension liquid obtained by suspending a rice bacterial grain rot bacterium (Burkholderia glumae MAFF301441) that had been cultured in a PPGA culture medium for 24 hours in distilled water (about $10^8$ cfu/ml), and left under a reduced pressure condition by a water flow pump for 11 hours to make contaminated seed rice.

The contaminated seed rice was mixed with healthy seed rice so that the contamination rate became 10%, and the mixture was immersed in each treatment liquid at 25°C for 48 hours. The mixture was then subjected to seed soaking at 25°C for 3 days by using distilled water, and forced sprouting at 32°C for 16 hours was then conducted.

About 50 particles of the sprouted seed rice were seeded on a balance dish (44 mm x 44 mm x 15 mm) in which a nursery soil for growing paddy rice seedlings had been filled, the seedlings were grown in a greenhouse, and the pathogenesis was examined at about ten days after the seeding.

The respective treatment liquids were made as follows. D-25 strain was subjected to shaking culture for 2 days at 25°C in a PPG liquid culture medium. This culture liquid was centrifuged to give a culture supernatant liquid. Furthermore, distilled water in the same amount as that of the removed supernatant was added to the fungus body obtained by the centrifugation, whereby a fungus body suspension liquid was obtained. The supernatant liquid and fungus body suspension liquid were used as the treatment liquids.

The examination on the pathogenesis was conducted on all of the seedlings, and an index (healthy seedling: 0, seedling with pathogenesis other than withering: 3, withered seedling: 5) was given depending on the degree of the pathogenesis, and the severity and preventive value were calculated according to the following formulas.

[Mathematical Formula 1]

Severity = {Σ (number of seedlings at each degree of pathogenesis × index)/(5 × number of examined seedlings)} × 100

[Mathematical Formula 2]

Preventive value = (1 – severity at treated region/severity at untreated region) × 100

The preventive value was 96.1 in the treatment with the fungus body suspension liquid of D-25 strain, and thus a very high pathogenesis-suppressing effect was observed. On the other hand, any pathogenesis-suppressing effect was not observed in the treatment with the culture supernatant liquid (Table 6 and Figs. 4(a) to 4(d)).

[Table 6]

Effects of suppressing pathogenesis of respective treatment liquids on rice bacterial grain rot (seedling rot)

| Strain or material | Number of examined seedlings (pieces) | Number of seedlings in each index of pathogenesis | | | Rate of diseased Seedlings (%) | Severity | Preventive value |
|---|---|---|---|---|---|---|---|
| | | 0 | 3 | 5 | | | |
| D-25 culture supernatant liquid | 115 | 15 | 8 | 92 | 88.4 | 85.9 | 14.1 |
| D-25 fungus body suspension liquid | 129 | 123 | 2 | 4 | 4.5 | 3.9 | 96.1 |
| Untreated | 92 | 0 | 0 | 92 | 100.0 | 100.0 | |

Industrial Applicability

According to the present invention, a bacterium that controls a disease damage by a pathogenic filamentous fungus, a pathogenic bacterium or a pathogenic virus in a host plant, a method for controlling a disease damage in a plant by using this bacterium, and a plant having a resistance against a disease damage which is made by this method are provided.

**Claims**

1. A method for controlling a disease damage by a pathogenic filamentous fungus, a pathogenic bacterium or a pathogenic virus in a plant, comprising a step of artificially infecting a plant with a bacterium that belongs to the genus Collimonas and has an ability to impart a resistance against a disease damage by a pathogenic filamentous fungus, a pathogenic bacterium or a pathogenic virus to a host plant by living in symbiosis in the body of the plant.

2. The method for controlling a disease damage by a pathogenic filamentous fungus, a pathogenic bacterium or a pathogenic virus in a plant according to claim 1, wherein the bacterium is a novel Collimonas bacterium (Accession No. NITE P-1104).

3. The method for controlling a disease damage by a pathogenic filamentous fungus, a pathogenic bacterium or a pathogenic virus in a plant according to claim 1 or 2, wherein the plant is a plant that belongs to Gramineae or Solanaceae.

4. An agent for controlling a disease damage by a pathogenic filamentous fungus, a pathogenic bacterium or a pathogenic virus in a plant, which contains, as an active ingredient, a bacterium that belongs to the genus Collimonas and has an ability to impart a resistance against a disease damage by a pathogenic filamentous fungus, a pathogenic bacterium or a pathogenic virus to a host plant by living in symbiosis in the body of the plant.

5. The controlling agent according to claim 4, wherein the bacterium is a novel Collimonas bacterium (Accession No. NITE P-1104).

6. The controlling agent according to claim 4 or 5, wherein the plant is a plant that belongs to Gramineae or Solanaceae.

7. A plant having a resistance against a disease damage by a pathogenic filamentous fungus, a pathogenic bacterium or a pathogenic virus, which has been artificially infected with a novel Collimonas bacterium (Accession No. NITE P-1104) that has an ability to impart a resistance against a disease damage by a pathogenic filamentous fungus, a pathogenic bacterium or a pathogenic virus to a host plant by living in symbiosis in the body of the plant.

FIG. 1

FIG. 2

FIG. 3 (a)

FIG. 3 (b)

FIG. 4

(a)

(b)

(c)

(d)

## INTERNATIONAL SEARCH REPORT

| | International application No. |
|---|---|
| | PCT/JP2013/056640 |

**A. CLASSIFICATION OF SUBJECT MATTER**
*A01N63/00*(2006.01)i, *A01P3/00*(2006.01)i, *A01G7/00*(2006.01)n, *C12N1/20* (2006.01)n

According to International Patent Classification (IPC) or to both national classification and IPC

**B. FIELDS SEARCHED**

Minimum documentation searched (classification system followed by classification symbols)
A01N63/00, A01P3/00, A01G7/00, C12N1/20

Documentation searched other than minimum documentation to the extent that such documents are included in the fields searched

| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)
CAplus/REGISTRY/BIOSIS(STN), JSTPlus/JMEDPlus/JST7580(JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>A | KAMILOVA et al., *Collimonas fungivorans*, an unpredicted *in vitro* but efficient *in vivo* biocontrol agent for the suppression of tomato foot and root rot, ENVIRONMENTAL MICROBIOLOGY, 2007, Vol. 9, P. 1597-1603, entire text | 1,3-4,6<br>2,5,7 |
| A | LEVEAU et al., The bacterial genus *Collimonas*: mycophagy, weathering and other adaptive solutions to life in oligotrophic soil environments, ENVIRONMENTAL MICROBIOLOGY, 2010, Vol. 12, P. 281-292, entire text, P. 285 'Antifungal and biocontrol activity' | 1-7 |

|X| Further documents are listed in the continuation of Box C.    | | See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>   13 May, 2013 (13.05.13) | Date of mailing of the international search report<br>   28 May, 2013 (28.05.13) |
|---|---|
| Name and mailing address of the ISA/<br>   Japanese Patent Office | Authorized officer |
| Facsimile No. | Telephone No. |

Form PCT/ISA/210 (second sheet) (July 2009)

**INTERNATIONAL SEARCH REPORT**

| International application No. |
| --- |
| PCT/JP2013/056640 |

C (Continuation). DOCUMENTS CONSIDERED TO BE RELEVANT

| Category* | Citation of document, with indication, where appropriate, of the relevant passages | Relevant to claim No. |
| --- | --- | --- |
| E,X | WO 2013/038542 A1 (New Environmental Technology Council), 21 March 2013 (21.03.2013), entire text; particularly, claims 1 to 4; examples 1 to 2 (Family: none) | 1-7 |
| E,X | WO 2013/038575 A1 (New Environmental Technology Council), 21 March 2013 (21.03.2013), entire text; particularly, claims 1 to 6; examples 1 to 9 (Family: none) | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WIESTE DE BOER ; JOHAN H. J. LEVEAU ; GEORGE A. KOWALCHUK ; PAULIEN J. A. KLEIN GUNNEWIEK ; EDWIN C. A. ABELN ; MARIAN J. FIGGE ; KLAAS SJOLLEMA ; JAAP D. JANSE ; JOHANNES A. VAN VEEN.** *Collimonas fungivorans gen. nov., sp. nov., a chitinolytic soil bacterium with the ability to grow on living fungal hyphae*
- **FRANCESCA MELA ; KATHRIN FRITSCHE ; WIETSE DE BOER ; JOHANNES A VAN VEEN ; LEO H DE GRAAFF ; MARLIES VAN DEN BERG ; JOHAN HJ LEVEAU.** *Dual transcriptional profiling of a bacterial/fungal confrontation: Collimonas fungivorans versus Aspergillus niger*

- **FAINA KAMILOVA ; JOHAN H. J. LEVEAU ; BEN LUGTENBERG.** *Collimonas fungivorans, an unpredicted in vitro but efficient in vivo biocontrol agent for the suppression of tomato foot and root rot*
- **SACHIE HOPPENER-OGAWA.** *Ecology of mycophagous Collimonas bacteria in soi*
- *pathogenic bacterium: Pseudomonus avenae Manns,* 1909